(19) Europäisches Patentamt
European Patent Office
Office européen des brevets

(11) **EP 3 592 216 B1**

(12) **EUROPÄISCHE PATENTSCHRIFT**

(45) Veröffentlichungstag und Bekanntmachung des Hinweises auf die Patenterteilung:
**11.05.2022   Patentblatt 2022/19**

(21) Anmeldenummer: **18750144.0**

(22) Anmeldetag: **31.07.2018**

(51) Internationale Patentklassifikation (IPC):
**A61B 5/00** (2006.01)       **A61B 5/022** (2006.01)
A61B 5/024 (2006.01)       G16H 50/20 (2018.01)

(52) Gemeinsame Patentklassifikation (CPC):
**A61B 5/02225; A61B 5/7264;** A61B 5/024;
A61B 5/7203; A61B 5/7225; A61B 5/7257;
A61B 2560/0475; G16H 50/20

(86) Internationale Anmeldenummer:
**PCT/EP2018/070701**

(87) Internationale Veröffentlichungsnummer:
**WO 2019/025427 (07.02.2019 Gazette 2019/06)**

(54) **VERFAHREN ZUM BETREIBEN EINER BLUTDRUCKMESSVORRICHTUNG**

METHOD FOR OPERATING A BLOOD PRESSURE MEASURING DEVICE

PROCÉDÉ PERMETTANT DE FAIRE FONCTIONNER UN DISPOSITIF DE MESURE DE LA TENSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.07.2017   DE 102017117337**

(43) Veröffentlichungstag der Anmeldung:
**15.01.2020   Patentblatt 2020/03**

(73) Patentinhaber: **Redwave Medical GmbH**
**07745 Jena (DE)**

(72) Erfinder:
• **DITTRICH, Verena**
**99084 Erfurt (DE)**
• **STOCKMANN, Chris**
**07607 Eisenberg (DE)**
• **MAINKA, Andreas**
**07747 Jena (DE)**

(74) Vertreter: **Kruspig, Volkmar**
**Meissner Bolte Patentanwälte**
**Rechtsanwälte Partnerschaft mbB**
**Berliner Straße 1**
**07545 Gera (DE)**

(56) Entgegenhaltungen:
WO-A1-92/03966       US-A- 5 533 511
US-A1- 2013 289 421

**Beschreibung**

Die Erfindung betrifft ein Verfahren zum Betreiben einer Blutdruckmessvorrichtung nach Anspruch 1 und die Verwendung eines solchen Verfahrens nach Anspruch 9. Oszillometrisch arbeitende Blutdruckmessvorrichtungen sind bekannt. Diese werden in der Regel so betrieben, dass zunächst eine Manschette um eine Extremität, z.B. Oberarm, gelegt wird. Die Manschette wird mittels einer Pumpe oder per Hand aufgepumpt. Durch den Druck der Manschette wird der Blutfluss in einem innerhalb der Extremität verlaufenden Gefäß unterbrochen. Der Druck in der Manschette wird sodann wieder abgelassen, sodass sich der Blutfluss in dem abgedrückten Gefäß wieder nachweisen lässt. Dabei ergibt sich der Blutdruck in diesem Blutgefäß durch den momentan in der Manschette herrschenden Druck. Mit einem solchen Verfahren lassen sich beispielsweise der mittlere arterielle Druck und daraus sowohl der systolische als auch der diastolische Blutdruck im Blutgefäß bestimmen.

Der systolische und der diastolische Blutdruck sind wesentliche Parameter, mit denen sich der Kreislaufzustand eines Probanden charakterisieren lässt. Diese Parameter reichen jedoch in vielen Fällen nicht aus. Sehr häufig lassen sich wirklich detaillierte Aussagen über die Hämodynamik eines Lebewesens nur dann treffen, wenn eine genauere Kenntnis der Gefäßeigenschaften vorliegt. Diese lassen sich jedoch aus einer einfachen Blutdruckmessung der bekannten Art nicht gewinnen, sondern dafür ist eine Analyse der Pulswellen notwendig.

Das Patent US 2017/0181648 A1 offenbart ein Verfahren, welches in der Lage ist, sehr robust die Blutdruckwerte im Oberarm zu bestimmen. Dafür werden zwei spezielle Pulswellen-Messeinheiten am oberen und unteren Ende der Manschette eingesetzt und die Pulswellensignale miteinander verrechnet. Eine Bestimmung der Gefäßeigenschaften und der Hämodynamik eines Lebewesens lassen sich, wie mit jedem anderen Standard-Blutdruckmessgerät, jedoch auch hiermit nicht durchführen.

Die Pulswellenanalyse hingegen enthält mit dem zentralen Blutdruck und der Pulswellengeschwindigkeit (PWV) Informationen über die Steifigkeit der arteriellen Gefäßwand und liefert Aussagen, die weit über jene hinausgehen, die von den am Oberarm gemessenen Blutdruckwerten, wie sie beispielweise bei Heimmessungen zur Anwendung kommen, abgeleitet werden können. Der zentrale Blutdruck und die PWV sind unabhängige Prädiktoren kardiovaskulärer Ereignisse (Herzinfarkt, Schlaganfall, Tod) und eine vielversprechende Möglichkeit, Risikopatienten, insbesondere solche mit intermediärem kardiovaskulären Risiko, differenziert bewerten zu können.

Der zentrale, in der Aorta gemessene Blutdruck hat in der Mehrzahl der Studien eine sehr viel größere prädiktive Wertigkeit als der periphere, am Oberarm gemessene Blutdruck. Hinzu kommt, dass neuere Daten in Form von Pilotstudien darauf hinweisen, dass sowohl eine Bluthochdruck- als auch eine Herzinsuffizienztherapie besser sind, wenn sie sich am zentralen Blutdruck orientieren und nicht am (konventionellen) Oberarm-Blutdruck.

Aus dem Stand der Technik bekannte Systeme, welche die Pulswellenanalyse unterstützen, sind allerdings teuer und komplex in der Anwendung und somit insbesondere für den Einsatz beim ungeübten Laien, aber auch für den breiten praktischen Einsatz ungeeignet. Darüber hinaus erfordert eine herkömmliche Pulswellenanalyse einen veränderten und verlängerten Messablauf, der sich insbesondere unter Heimbedingungen nicht kontrolliert realisieren lässt.

Gemäß den hierzu üblichen Verfahren wird ein spezieller Zieldruck, d.h. ein Druck-Plateau angefahren und für mehrere Sekunden, üblicherweise 10 Sekunden, gehalten. Eine solche Messmethodik ist nicht nur unangenehm für den Patienten, sondern der zusätzliche Messaufwand erhöht außerdem die Fehleranfälligkeit beispielsweise durch Bewegungsartefakte. Diese methodischen Anforderungen stellen besondere Anforderungen an die Geräte, die für die Messung zum Einsatz kommen. Es müssen spezielle Geräte entwickelt werden, welche komplex und mit erheblichen Kosten verbunden sind. Die für die Messung eingesetzten Algorithmen sind dabei ausschließlich mit der jeweils verwendeten Hardware kompatibel und können nicht auf anderen Plattformen eingesetzt werden.

Das Patent DE 10 2004 011 779 B4 offenbart u.a. eine Anordnung, welche ein Standard-Blutdruckmessgerät beschreibt. Diese wird so oder ähnlich eingesetzt um insbesondere die Wellenform der einzelnen Pulswellen miteinander zu vergleichen und somit Störungen und Artefakte sicher zu erkennen. Eine solche Anordnung ist kostengünstig, robust und deshalb weltweit verbreitet und findet in der Mehrzahl der Standard-Blutdruckmessgeräte Anwendung.

Ein weiteres sehr großes Problem ist, dass bei der Durchführung einer Pulswellenanalyse lediglich eine einzelne Transferfunktion für die gesamte Population eingesetzt wird, womit eine deutliche Unschärfe und Qualitätseinbußen einhergehen. Darüber hinaus bietet diese Technologie nur wenig Informationen über weitere hämodynamische Parameter, da lediglich die Pulswellen auf dem konstanten Druckniveau ausgewertet werden.

Aus der US 2013/289421 A1 ist eine Anordnung zum Messen des Druckes in einem Blutgefäß vorbekannt, enthaltend eine Manschette zum Anlegen um eine Extremität, eine Pumpe zum Aufpumpen der Manschette, einen Drucksensor zum Registrieren des in der Manschette anliegenden zeitlichen Druckverlaufes sowie eine Steuer- und Speichereinheit, welche dem Betreiben der Pumpe und des Drucksensors dient und die den fertigen Druckverlauf abspeichert.

Zusätzlich weist die Steuer- und Speichereinheit ein Steuer- und Auswerteprogramm für ein Abseparieren pulsförmiger Anteile im zeitlichen Druckverlauf während einer Aufpump- und/oder Ablassphase des zeitlichen Druckverlaufes auf. Das Programm ermöglicht darüber hinaus eine Signalanalyse der abseparierten pulsförmigen Anteile.

Aus der Patentschrift US 5533511 A ist ein Verfahren zur Blutdruckmessung mit der Klassifizierung des Signals im Zeitraum und der anschließenden Fast FourierTransformation zur Bestimmung hämodynamischer Parameter bekannt. Hierbei wird jedoch nicht auf ein oszillometrisches Signal zurückgegriffen; ein manschettenbasiertes Blutdruckmessgerät wird lediglich zur Kalibrierung verwendet.

Aus der WO 92/03966 A1 ist außerdem die Analyse eines oszillometrischen Signals mittels eines neuronalen Netzwerks bekannt.

Es besteht daher die Aufgabe, ein Verfahren anzugeben, mit dem Eigenschaften aortaler Pulswellen mit einem minimalen Aufwand ermittelt werden können, wobei das gesuchte Verfahren eine vollständige Pulswellenanalyse mit zusätzlichen hämodynamischen Parametern ermöglichen soll. Damit soll es ermöglicht werden, die Datengrundlage der Pulswellenanalyse so zu vereinfachen, robust zu gestalten und zu erweitern, dass die Pulswellenanalyse der breiten Masse der niedergelassenen Ärzte und insbesondere den Patienten zuhause einfach und kostengünstig zur Verfügung gestellt werden kann.

Die Lösung der Aufgabe erfolgt mit einem Verfahren zum Betreiben einer Blutdruckmessvorrichtung mit den Merkmalen des Anspruchs 1 und einer Verwendung gemäß Anspruch 9. Die Unteransprüche enthalten zweckmäßige bzw. vorteilhafte Ausführungsformen des Verfahrens bzw. der Vorrichtung.

Das Verfahren erfolgt erfindungsgemäß mit folgenden Verfahrensschritten:
Es erfolgt in einem ersten Schritt ein Ausführen einer Blutdruckmessung mit der Blutdruckmessvorrichtung, bestehend aus einer Manschette zum Umlegen um eine Extremität, einer Pumpe zum Aufpumpen der Manschette und einem Drucksensor zum Registrieren des Drucks innerhalb der Manschette. Dabei erfolgt ein Aufpumpen der Manschette in einer Aufpumpphase und ein Ablassen des Drucks in der Manschette in einer Ablassphase.

Während der Aufpumpphase und/oder während der Ablassphase erfolgt ein Registrieren und Speichern des zeitlichen Druckverlaufs in der Manschette in einer Steuer- und Speichereinheit. Es erfolgt danach ein Extrahieren eines pulsartigen Signalanteils aus dem zeitlichen Druckverlauf des gemessenen Manschettendrucks während der Aufpumpphase und/oder der Ablassphase durch eine Signalanalyse innerhalb der Steuer- und Speichereinheit in Verbindung mit einer Signalanalyse des extrahierten pulsartigen Signalanteils.

Die Signalanalyse erfolgt in der Steuer- und Speichereinheit mit folgenden Schritten:
In einem ersten Schritt erfolgt ein Zerlegen des pulsartigen Signalanteils in Daten über einzelne Pulswellen. Die Daten der jeweiligen einzelnen Pulswellen werden dann aus Werten mindestens einer Berechnungsfunktion aus einer vorgegebenen, ggf. auch flexibel und/oder autonom erweiterungsfähigen, Berechnungsfunktionsmenge zum Erzeugen einer jeweiligen klassifizierten Pulswelle kombiniert.

Es erfolgt sodann ein Transformieren der jeweiligen klassifizierten einzelnen Pulswelle. Die Transformation in ein pulswellenspezifisches Amplituden- und Phasenspektrum für jede klassifizierte Einzelperiode hat sich als geeignet herausgestellt, ist jedoch nicht darauf beschränkt. Abschließend erfolgt ein Auswerten der transformierten Daten zum Ermitteln hämodynamischer Parameter.

Grundsätzlich wird bei dem erfindungsgemäßen Verfahren zunächst auf das bekannte Verfahren der gewöhnlichen Blutdruckmessung zurückgegriffen. Im Gegensatz zur herkömmlichen Blutdruckmessung, bei der über die Manschette ein arterieller Blutfluss gestoppt und bei einem dosierten Ablassen des Drucks aus der Manschette wieder nachgewiesen wird, wird aber erfindungsgemäß der Druck in der Manschette während der gesamten Aufpumpphase und/oder der gesamten Ablassphase in seinem Zeitverlauf registriert. Von diesem aufgezeichneten Zeitverlauf in einer der beiden oder in beiden Phasen wird eine pulsartige Komponente extrahiert. Diese am Arm oder einer anderen Extremität gemessene pulsartige Komponente gibt dann nach weiterer Signalanalyse zunächst eine Information über einzelne Pulswellen vom Arm oder einer anderen Extremität und schließlich über die jeweiligen aortalen Pulswellen des Probanden. Im Gegensatz zu den sonst bekannten Messverfahren für aortale Pulswellen, genügt erfindungsgemäß die übliche Vorgehensweise bei einer Blutdruckmessung, um die aortale und somit im Körperinneren erzeugte und vorherrschende Pulswelle mit einem minimalen Geräteaufwand und einer minimierten Belastung des Probanden zu detektieren und zu analysieren.

Ein weiterer erfindungsgemäßer Aspekt ist eine komplexere Signalanalyse des pulsartigen Signalanteils, der mit einer Formanalyse jeder einzelnen Periode im gesamten pulsartigen Signalanteil verknüpft ist. Hiermit sind Informationen über eine Liste von Einzelperioden verfügbar, die einen tieferen Einblick in die Datenmenge des pulsförmigen Signalanteils erlauben. Zusätzlich wird außerdem jeder einzelne Puls, d.h. der Signalverlauf in jeder Einzelperiode in seiner Form klassifiziert. Dies erfolgt über ein Kombinieren des Signalverlaufs jeder Einzelperiode aus vorgegebenen, ggf. auch flexibel und/oder autonom erweiterungsfähigen, Berechnungsfunktionen. Im Gegensatz zur herkömmlichen Detektion und Analyse aortaler Pulswellen wird somit eine Transformation nicht nur mit Hilfe einer Berechnungsfunktion, sondern mehrerer Berechnungsfunktionen vorgenommen. Es werden hierdurch Details des Signalverlaufs gleichzeitig ermittelt, objektiv klassifiziert und, nach einer Transformation in beispielsweise das periodenspezifische Amplituden- und Phasenspektrum, dessen Eigenschaften als Kenngrößen ausgegeben.

In einer ersten Ausführungsform erfolgt das Zerlegen des pulsartigen Signalanteils in Daten über Einzelperioden. Eine Fußpunktdetektion im zeitlichen Verlauf des pulsartigen Signalanteils hat sich als geeignet herausgestellt, ist jedoch nicht darauf beschränkt. Hierdurch können tatsächliche Periodenverläufe und Grenzen der Einzelperioden zuverlässig erkannt werden.

Bei einer Ausgestaltung des Verfahren werden bei dem Kombinieren der Daten der jeweiligen Einzelperioden zum Erzeugen der jeweiligen klassifizierten Einzelperiode mindestens zwei Berechnungsfunktionen gewichtet miteinander kombiniert. Hierdurch ist es möglich, Signalverläufe von Einzelperioden nicht nur einer Berechnungsfunktion zuzuordnen, sondern auch Zwischenzuordnungen zu mehreren Berechnungsfunktionen ausführen zu lassen

Das Transformieren der jeweiligen klassifizierten Einzelperiode erfolgt bei einer Ausführungsform des Verfahrens mittels einer Fourier-Analyseeinheit in das periodenspezifische Amplituden- und Phasenspektrum. Die Fourier-Analyseeinheit kann auch im Form eines Auswerteprogramms realisiert sein.

Bei einer Ausgestaltung des Verfahrens erfolgt eine Rücktransformation des periodenspezifischen Amplituden- und Phasenspektrums der klassifizierten einzelnen Pulswelle zum Bestimmen des Zeitverlaufs der aortalen Pulswelle. Durch die Rücktransformation erhält man die aortale Pulskurve dieser spezifischen Klasse und Ergebnisse zu hämodynamischen Parametern der Pulswellenanalyse.

Bei einer Ausführungsform erfolgt das Registrieren des zeitlichen Druckverlaufs in der Aufpumpphase und/oder in der Ablassphase digital mit einer festen gerätespezifischen Abtastrate und einer festen gerätespezifischen Auflösung, wobei ein anschließendes Sampeln auf eine plattformunabhängige Abtastrate in Verbindung mit einer Approximation des gemessenen Zeitverlaufs des Manschettendrucks erfolgt. Hierdurch ist es möglich, die erhaltenen Druckverläufe plattformunabhängig, und damit Geräte-unabhängig, auszuwerten und zu vergleichen.

Bei einer Ausgestaltung des Verfahren erfolgt bei dem Extrahieren des pulsförmigen Signalanteils über einen Bandpass-Filter ein Abseparieren eines nichtoszillierenden Manschettendrucks. Der nichtoszillierende Manschettendruck gibt zwar keinen Aufschluss über die Pulswelle als solche, er ermöglicht aber einen Rückschluss auf mögliche unerwünschte und verfälschende Einflüsse auf den Messvorgang.

Bei einer Ausgestaltung des Verfahren wird der abseparierte nichtoszillierende Manschettendruck auf Übereinstimmung mit einem Normverlauf geprüft, wobei aus dem Grad der Abweichung des nichtoszillierenden Pumptrends von dem linearen Verlauf ein Bestimmen eines Artefaktparameters für ein Anzeigen einer messwertverfälschenden Beeinflussung erfolgt. Der Artefaktparameter ist ein Maß für die Qualität der Signale und trägt Informationen über die Untersuchung. Er beschreibt, welchem verfälschenden Einfluss der gesamte Ablauf der Druckmessung ausgesetzt gewesen ist und wie valide die berechneten Ergebnisse sind.

Die im Folgenden beschriebene Anordnung dient der Durchführung des erfindungsgemäßen Verfahrens, ist jedoch als solche nicht Gegenstand der vorliegenden Erfindung.

Eine Anordnung zum Messen des Druckes in einem Blutgefäß enthält eine Manschette zum Anlegen um eine Extremität, eine Pumpe zum Aufpumpen der Manschette, einen Drucksensor zum Registrieren des in der Manschette anliegenden zeitlichen Druckverlaufs und eine Steuer- und Speichereinheit zum Betreiben der Pumpe und des Drucksensors und zum Speichern des zeitlichen Druckverlaufs. Dabei weist die Steuer- und Speichereinheit ein Steuer- und Auswerteprogramm für ein Abseparieren pulsförmiger Anteile im zeitlichen Druckverlauf während einer Aufpump- und/oder Ablassphase des zeitlichen Druckverlaufs und für eine Signalanalyse der abseparierten pulsförmigen Anteile auf.

Das Steuer- und Auswerteprogramm kann bei einer Ausgestaltung der Vorrichtung von einem externen Speichermittel über eine Schnittstelle auf die Steuer- und Speichereinheit aufspielbar und aktualisierbar sein. Die Steuer- und Speichereinheit lässt sich hierdurch in ihrer Betriebsweise leicht neu konfigurieren.

Bei einer weiteren Ausführungsform weist die Steuer- und Speichereinheit eine Schnittstelle zum Auslesen gespeicherter Daten, insbesondere des gemessenen zeitlichen Druckverlaufs, an eine externe Auswerteeinheit und/oder einen fernen Host auf. Hierdurch ist eine externe, ferne und zentralisierte Auswertung sowie auch eine Fernüberwachung möglich.

Bei einer Ausführungsform weist die Steuer- und Speichereinheit ein Display zur Ausgabe von aus den pulsförmigen Anteilen des Druckverlaufs gewonnen Parametern, insbesondere zur Ausgabe einer Herzschlagfrequenz und/oder hämodynamische Parameter aus der Pulswellenanalyse und/oder einer Verlaufsform einer aortalen Pulswelle, auf.

Bei einer weiteren Ausführungsform weist die Steuer- und Speichereinheit eine Umschaltmöglichkeit zwischen einem ersten Betriebsmodus zum Ausführen einer Standard-Blutdruckmessung und einem zweiten Betriebsmodus zum Ausführen einer Messung der pulsförmigen Anteile des zeitlichen Druckverlaufs und zum Ermitteln von Parametern einer aortalen Pulswelle auf. Die Blutdruckmessvorrichtung kann dadurch in verschiedenen Betriebsarten betrieben werden.

Das genannte Verfahren sowie die Anordnung werden zum Ermitteln und Analysieren mind. einer aortalen Pulswelle und zur Bestimmung hämodynamischer Parameter der Pulswellenanalyse, wie z.B. aortaler Blutdruck, verwendet.

Das Verfahren und die Anordnung sollen nachfolgend anhand von Ausführungsbeispielen näher erläutert werden. Zur Verdeutlichung dienen die beigefügten Figuren.

Es zeigt:

Fig. 1 eine Grundkonfiguration einer Anordnung zum Ausführen des Verfahrens,

Fig. 2 einen beispielhaften Verlauf des Druckes innerhalb der Manschette mit einer Aufpump- und einer Ablassphase,

Fig. 3 eine beispielhafte Darstellung des Amplitudenspektrums der Impulsantwort für einen beispielhaften Bandpassfilter,

Fig. 4 eine beispielhafte Darstellung des Phasenspektrums der Impulsantwort für einen beispielhaften Bandpassfilter,

Fig. 5 eine beispielhafte Darstellung eines in oszillierende und nicht oszillierende Komponenten zerlegten Drucksignals,

Fig. 6 eine beispielhafte Darstellung einer Fußpunktbestimmung an einer einzelnen Pulswelle,

Fig. 7 eine beispielhafte Pulswellenfolge mit erfolgter Fußpunktdetektion,

Fig. 8 eine beispielhafte Darstellung des relevanten Pulswellenfolgebereichs,

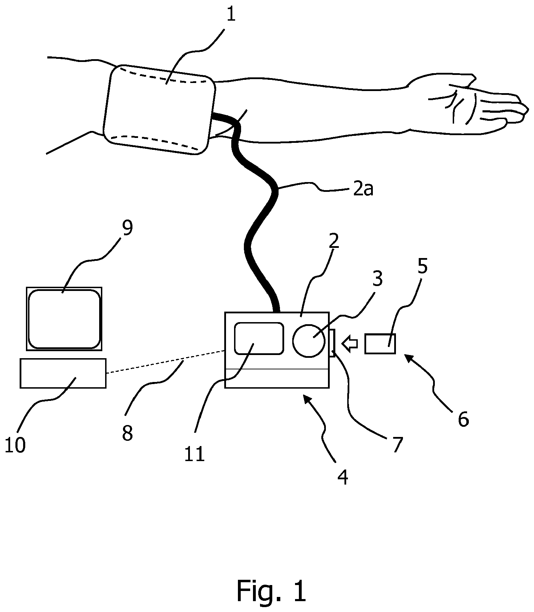

Fig. 1 zeigt eine Grundkonfiguration für eine Anordnung zum Ausführen des Verfahrens. Die Anordnung beinhaltet eine Manschette 1, die über eine Pumpe 2 über einen Schlauch 2a aufgepumpt werden kann. Die Manschette wird um eine Extremität, beispielsweise um einen Oberarm, herum angelegt. Weiterhin ist ein Drucksensor 3 vorgesehen, mit dem der zeitliche Verlauf des Druckes in der Manschette registriert wird. Der Drucksensor kann sich an einer beliebigen Position im Bereich des Systems von Manschette oder Pumpe befinden.
In der hier vorliegenden Konfiguration sind der Drucksensor und die Pumpe in einem Gehäuse zusammen mit weiteren Speicher- und Datenverarbeitungselementen und einer Steuer- und Speichereinheit 4 vereinigt. Die Steuer- und Speichereinheit steuert sowohl den Betrieb der Pumpe als auch die Messwerterfassung durch den Drucksensor. Die Pumpe realisiert insbesondere unter der Steuerung der Steuer- und Verarbeitungseinheit einen z.B. zeitlich linearen Druckanstieg in der Manschette während einer Aufpumpphase. Während einer Ablassphase wird über ein hier nicht gezeigtes Auslassventil bspw. ein zeitlich lineares Abfallen des Drucks in der Manschette bewirkt. Diese linearen Druckverläufe sind grundsätzlich nicht notwendig, erweisen sich jedoch als zweckmäßig und vorteilhaft im Hinblick auf eine spätere Artefaktdetektion.
Die Steuer- und Speichereinheit 4 beinhaltet ein Steuer- und Auswerteprogramm 5, das die weiter unten genauer dargestellten Auswerteschritte ausführt. Das Steuer- und Auswerteprogramm liegt im hier vorliegenden Beispiel gespeichert auf einem externen Speichermittel 6, beispielsweise einer SD-Karte, einem USB-Stick oder einem anderen Speichermittel vor. Es kann über eine Schnittstelle 7, beispielsweise einen SD-Schacht oder einen USB-Anschluss, in die Steuer- und Speichereinheit 4 geladen werden. Möglich ist natürlich auch eine permanente Speicherung des Steuer- und Auswerteprogramms in der Steuer- und Speichereinheit nach Art einer festen oder auch über die Schnittstellen aktualisierbaren Firmware.
Die Steuer- und Speichereinheit 4 enthält eine weitere Schnittstelle 8 zum Auslesen der gespeicherten Messdaten und zum Übergeben der Daten an eine externe Auswerteeinheit 9, beispielsweise einen PC. Die externe Auswerteeinheit weist ein Auswerteprogramm 10 auf, mit dem die gespeicherten ausgelesenen Daten ebenfalls analysiert werden können. Die Schnittstelle 8 kann sowohl kabellos als auch drahtgebunden ausgebildet sein. Möglich ist auch eine Datenübertragung über ein Kommunikationsnetz an einen fernen Host, sodass hier eine Fernüberwachung und Fernauswertung von Blutdruckparametern möglich ist.
In dem hier vorliegenden Fall enthält die Steuer- und Speichereinheit 4 außerdem ein Display 11 zur direkten Ausgabe der gemessenen Blutdruckdaten und des zeitlichen Druckverlaufs in der Manschette 1. Es können weitere Bedienelemente vorgesehen sein, insbesondere zum Umschalten zwischen einer konventionellen Blutdruckmessung und einer Betriebsart, bei der das erfindungsgemäße Verfahren ausgeführt wird.
Wie erwähnt, ist es einer der wesentlichen Aspekte des erfindungsgemäßen Verfahrens, dass die Blutdrucksignale bei der Messung beim Aufpumpen und/oder Ablassen der Manschette in ihrem zeitlichen Verlauf sowohl aufgezeichnet als auch ausgewertet und für deren spätere Auswertung auch transformiert werden. Die Apparatur besteht dabei wie beschrieben aus einer Manschette, einem Sensor und einer Pumpe. Durch die Pumpe wird die Manschette auf einen bestimmten Druck aufgepumpt. Gleichzeitig werden dabei die Pulswellen während des Vorgangs des Aufpumpens und/oder des Ablassens einer Manschette erfasst, indem der Druckverlauf innerhalb der Manschette zeitlich verfolgt wird.
Mit Hilfe spezieller und nachfolgend näher beschriebener Verfahrensschritte werden sodann aus dem zeitlich erfassten Druckverlauf während der Aufpumpphase und/oder der Ablassphase am Arm oder einer anderen Extremität die für die Pulswellenanalyse erforderlichen Pulswellen extrahiert. Die Signale der extrahierten Pulswellen werden nach einer anschließenden Aufbereitung zu Werten transformiert, die über die aortalen Pulswellen genaueren Aufschluss ergeben. Störungen innerhalb des Signals und Artefakte der Messungen werden dabei erkannt und aus der Analyse ausgeschlossen und/oder entsprechend auskorrigiert oder auch signalisiert.

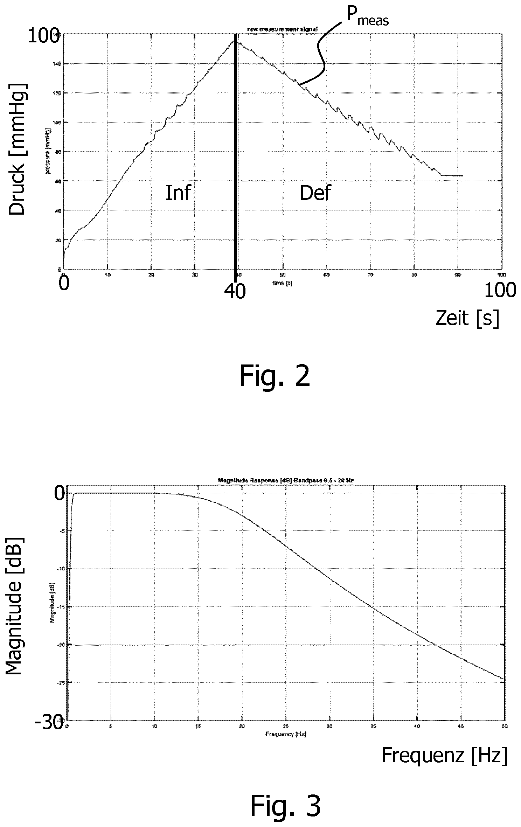

Fig. 2 zeigt einen beispielhaften zeitlichen Signalverlauf des aufgezeichneten Druckes in der Manschette gemäß des hierfür vorgesehenen Verfahrensablaufs. Es ist zu erkennen, dass der Druck in einer anfänglichen Aufpumpphase Inf zunächst ansteigt und dann in einer sich anschließenden Ablassphase Def wieder abfällt.

Es ist zu erkennen, dass der Druck in der Aufpumpphase im Wesentlichen linear ansteigt und in der Ablassphase im Wesentlichen linear abfällt. Sowohl der Verlauf des linearen Druckanstiegs als auch der Verlauf des linearen Druckabfalls ist von einer oszillatorischen Signalkomponente überlagert, die sich hier in Form von Zacken im Druckverlauf zeigt. Diese oszillatorische Signalkomponente beinhaltet die Information über die aortale Pulswelle. Sie wird erfindungsgemäß aus dem zeitlichen Druckverlauf extrahiert, wobei die nachfolgend erläuterten beispielhaften Verfahrensschritte angewendet werden.

Zum Aufzeichnen des in Fig. 2 gezeigten zeitlichen Druckverlaufs wird zunächst eine an sich übliche herkömmliche Blutdruckmessung durchgeführt. Im Unterschied zu der herkömmlichen Blutdruckmessung, bei der bspw. lediglich die maximale Amplitude der oszillierenden Signalkomponente registriert wird, wird bei dem erfindungsgemäßen Verfahren jedoch der zeitliche Druckverlauf $P_{meas}$ als Ganzes erfasst. Diese Messwerterfassung kann insbesondere digital erfolgen. Die Abtastrate und Auflösung ist dabei geräteabhängig. Dabei kommt beispielsweise eine Abtastrate von 40 Hz bei einer Auflösung von 12 Bit zur Anwendung.

Sowohl die Abtastrate als auch die Auflösung sind an sich nicht festgelegt, sondern ausschließlich gerätespezifische Größen. Zur Gewährleistung einer Plattformunabhängigkeit und somit einer bestmöglichen Verarbeitbarkeit wird die Messwertmenge des zeitlichen Druckverlaufs anschließend entweder in der Steuer- und Speichereinheit selbst oder in der externen Auswerteeinheit auf einen einheitlichen Standard so transformiert, dass dessen Verarbeitung plattformunabhängig und damit vereinheitlicht erfolgen kann. Hierzu kann das Signal des zeitlichen Druckverlaufs beispielsweise auf eine Abtastfrequenz von 100 Hz hochgesampelt werden. Für diesen Samplingprozess können verschiedene Approximationsverfahren zur Anwendung kommen. Insbesondere wird hierzu z.B. die sogenannte kubische Splineapproximation eingesetzt. Bei dieser Approximation werden zwei benachbarte Samplepunkte approximiert.

Für die kubische Splineapproximation werden für zwei benachbarte Samplepunkte $(x_i, y_i)$ und $(x_{i+1}, y_{i+1})$ Polynome vom Grad 3 ermittelt:

$$s_i(x) = a_i + b_i(x - x_i) + c_i(x - x_i)^2 + d_i(x - x_i)^3$$

Dabei gilt wegen der Stetigkeit des zu sampelnden Signals die Stetigkeitsforderung $s_i(x_{i+1}) = s_{i+1}(x_{i+1})$, wobei s zweimal stetig differenzierbar sein muss:

$$s_i'(x_{i+1}) = s_{i+1}'(x_{i+1})$$

$$s_i''(x_{i+1}) = s_{i+1}''(x_{i+1})$$

Bei der Wahl eines neuen $\Delta x$ ist eine Neuabtastung des Signals durchführbar. Wird das Ausgangssignal auf eine Abtastfrequenz von 100 Hz hochgesampelt, so gilt in diesem Fall somit:

$$\Delta x = \frac{1}{100}$$

Die Wahl der Art des Splinetyps bezüglich der Ränder spielt allerdings keine ausschlaggebende Rolle.

Aus diesem plattformunabhängig gesampelten Signal werden im nächsten Schritt die Pulswellen, das heißt der oszillatorische Anteil $P_{osc}$ des gemessenen zeitlichen Druckverlaufs $P_{meas}$ extrahiert. Die Extraktion erfolgt beispielsweise durch die Anwendung eines IIR-Bandpass-Filters. Ebenfalls kann ein FIR-Filter zur Anwendung kommen. Möglich ist hier ein Butterworth-Filter 6. Ordnung.

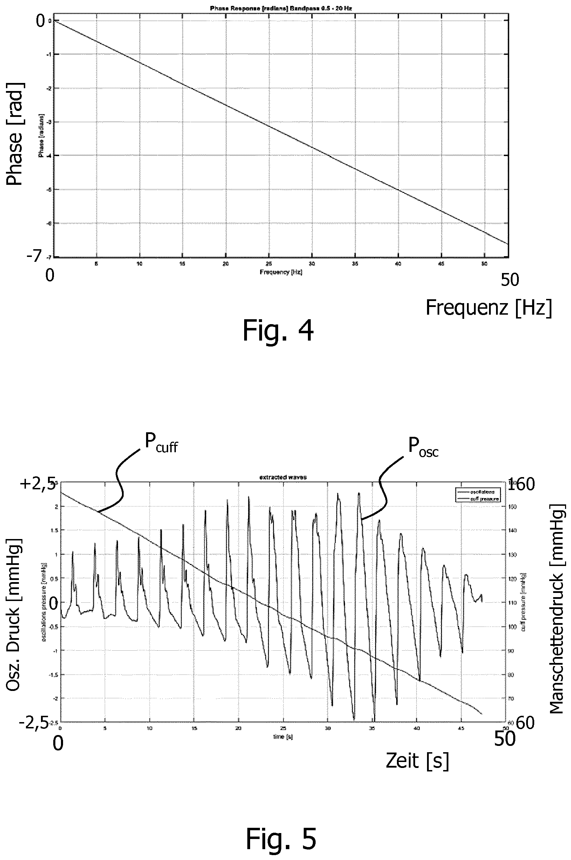

Die Figuren 3 und 4 zeigen hier beispielhafte Filtercharakteristiken. Fig. 3 zeigt die in dB angegebene Magnitude des Filters in Abhängigkeit von der Frequenz im Bereich von 0 bis 50 Hz. Fig. 4 zeigt einen beispielhaften Phasenverlauf des Bandpassfilters im Frequenzbereich von 0 bis 50 Hz. Der Bandpass-Bereich erstreckt sich dabei auf einen Frequenzbereich von 0,5 Hz bis 20 Hz. Er ist dabei bereits auf zu erwartende Frequenzen des pulsierenden Signalanteils angepasst. In Fig. 3 ist zu entnehmen, dass die Magnitude der Filterkennlinie in diesem Bereich quasi horizontal verläuft und erst bei größeren Frequenzen von mehr als 20 Hz abfällt. Der Filter besitzt die Eigenschaft eines Nullphasenfilters, um Verfälschungen des Phasenspektrums des verarbeiteten Signals zu vermeiden.

Für das gemessene zeitliche Drucksignal kann eine additive Beziehung angesetzt werden. Der gemessene zeitliche Druckverlauf ist dabei die Summe aus dem über die Pumpe erzeugten Manschettendruck und dem durch die Pulswelle erzeugten pulsförmigen Druck. Es gilt somit:

$$P_{meas} = P_{cuff} + P_{osc}$$

$P_{meas}$ ist der vom Drucksensor 3 gemessene zeitliche Druckverlauf, $P_{cuff}$ ist der von der Pumpe 2 erzeugte Pumpendruck, d.h. der Manschettendruck, und $P_{osc}$ sind die von der Pulswelle am Arm oder einer anderen Extremität erzeugten Druckoszillationen, aus denen letztlich die aortalen Pulswellen in ihrer Charakteristik bestimmt werden sollen.

Der zu verwendende Bandpassbereich für den Bandpassfilter lässt sich aus folgenden Überlegungen ableiten. Entsprechend andere Einsatzbedingungen bedingen damit selbstverständlich eine davon abweichende Grundkonfiguration. Die Messung findet am Probanden z.B. in Ruhe und einem hinreichend definierten physiologischen Zustand statt. Unter diesen Bedingungen kann von eindeutig bestimmbaren Herzraten im Bereich von 40 bis 100 Schlägen pro Minute ausgegangen werden. Daraus ergeben sich für den oszillatorischen Anteil $P_{osc}$ im zeitlichen Druckverlauf entsprechend definierte Grenzfrequenzen.

Wenn Hr_Low die niedrige Herzrate von 40 Schlägen pro Minute und Hr_High die hohe Herzrate von 100 Schlägen pro Minute ist, so ergeben sich daraus eine untere Grenzfrequenz $f_L$ und eine obere Grenzfrequenz $f_H$ durch $f_L$ = Hr_Low/60 = 40/60 ≈ 0,7 Hz bzw. $f_H$ = Hr_High/60 = 100/60 ≈ 1,7 Hz.

Die Energie einer durch das schlagende Herz erzeugten Pulswelle verteilt sich dabei über ein gewisses Frequenzspektrum. So liegt beispielsweise der größte Anteil der Energie der Pulswelle bei einer Herzrate von 60 Schlägen pro Minute im Bereich von 1-10 Hz, verteilt auf 10 harmonische Schwingungen.

Wird also ein Bereich von 10 harmonischen Schwingungen angenommen und werden dabei die oberen Grenzen berücksichtigt, so ergibt sich für die Pulswelle ein Frequenzbereich von 0,7 Hz bis 10·1,7 Hz, also von 0,7 bis 17 Hz. Diese Filtergrenzen sind allerdings nicht scharf ausgeprägt. Es wird daher jeweils beidseitig im Frequenzintervall ein Zusatzintervall hinzugefügt, so dass für den Bandpassfilter als Frequenzintervall zweckmäßigerweise ein Bereich von 0,5 - 20 Hz gewählt wird.

Der oszillatorische Anteil $P_{osc}$ des gemessenen zeitlichen Drucksignals $P_{meas}$, und damit die aus dem gemessenen Signal extrahierte Pulswelle, ergibt sich somit als eine Anwendung der Filterfunktion des Bandpassfilters auf das gemessene Drucksignal:

$$P_{osc} = filter(P_{meas})$$

Wird durch den Einsatz des Filters die Pulswelle extrahiert, folgt aus der obigen additiven Beziehung, dass der verbleibende Rest des ursprünglich aufgenommenen Drucksignals somit der von der Pumpe erzeugte Manschettendruck ist.

$$P_{cuff} = P_{meas} - P_{osc}$$

Das gemessene Drucksignal $P_{meas}$ ist damit in den Manschettendruck $P_{cuff}$ und die Pulsoszillationen $P_{osc}$ zerlegt worden.

Fig. 5 zeigt beispielhaft den aus dem Ausgangssignal gewonnenen oszillatorischen Anteil $P_{osc}$. Der Manschettendruck $P_{cuff}$ kann dann beispielsweise im Bereich der Aufpumpphase idealerweise einen nicht oszillierenden annähernd linearen Anstieg und im Bereich der Ablassphase einen nicht oszillierenden und zumindest annähernd linearen Abfall aufweisen, sofern die Pumpe eine zeitlich lineare Druckzunahme in der Manschette erzeugt und sofern beim Ablassen ein zeitlich linearer Druckabfall bewirkt wird. Fig. 5 zeigt, dass diese Anforderungen in dem hier vorliegenden Beispiel hinreichend gut erfüllt sind.

Ist die Charakteristik des Aufpumpens bzw. Ablassens, d.h. die von der Pumpe realisierte zeitliche Kennlinie des Manschettendrucks beim Aufpumpen bzw. die vom Ablassventil realisierte zeitliche Kennlinie des Manschettendrucks beim Ablassen, im Vorfeld bekannt, so können diese Kennlinien anhand der abseparierten Manschettendruck-Funktion $P_{cuff}$ auf ihre Genauigkeit überprüft werden. Es bietet sich hier somit die Möglichkeit einer Eichung der Messanordnung oder einer Ermittlung zusätzlicher Artefakte im Messprozess.

Ist z.B. die lineare Kennlinie des linearen Abfalls des Manschettendrucks vorab bekannt, so kann der real gemessene und abseparierte Manschettendruck $P_{cuff}$ auf Linearität untersucht werden. Starke Abweichungen des Manschettendrucks $P_{cuff}$ von der Linearität können dann als Bewegungsartefakte oder sonstige verfälschende Einflüsse auf den Messprozess gedeutet werden. Hierdurch ergibt sich die Möglichkeit, ein Fehlersignal zu gewinnen, das entsprechend an der Messanordnung signalisierend ausgegeben werden kann. Entsprechend können auch andere nicht lineare Kennlinien zur Auswertung von Artefakten bzw. eines verbleibenden Residuums herangezogen werden.

Mögliche Verfahren für die Bestimmung des Residuums und eine vergleichende Prüfung zwischen der vorab bekannten Kennlinie und dem zeitlichen Verlauf des gemessenen Manschettendruckes $P_{cuff}$ sind Abstandsbestimmungen der Werte des Manschettendrucks $P_{cuff}$ zur Regression der Funktion auf ein bekanntes Polynom n-ten Grades. Bei einem Test auf Linearität ist das Polynom entsprechend ein Polynom vom Grad 1.

Der Manschettendruck $P_{cuff}$ wird dabei in einem Schritt einer Regression unterworfen. In einem zweiten Schritt wird dann die daraus gewonnene Regressionsbeziehung regression($P_{cuff}$) mit einem Polynom n-ten Grades verglichen. Der Parameter des Artefakts bestimmt sich dann aus der folgenden Beziehung:

$$artefact = \left| regression\left(P_{cuff}\right) - p^n \right| > threshold$$

mit $p^n$ als einem Vergleichspolynom n-ten Grades. Das bedeutet, dass genau dann ein Messartefakt vorliegt, wenn der Betrag der Differenz zwischen der Regressionsbeziehung des Manschettendrucks $P_{cuff}$ zum Vergleichspolynom $p^n$ einen bestimmten vorgegebenen Schwellwert überschreitet. In einem solchen Fall wird von der Steuerungs- und Speichereinheit zum Beispiel ein Fehlersignal ausgegeben. Das verbliebene Residuum kann darüber hinaus weitere, wertvolle Informationen über die Untersuchung und Qualität der Signale liefern.

Im Anschluss an die Separation des pulsartigen Signalanteils $P_{osc}$ vom Manschettendruck $P_{cuff}$ erfolgt eine Auswertung des Pulswellensignals als solches. Die dabei ausgeführten Teilschritte sind zunächst das Zerlegen des pulsartigen Signalanteils in Einzelperioden und eine nachfolgende Analyse jeder einzelnen, mind. jedoch einer Einzelperiode selbst, denn die hier enthaltenen Daten repräsentieren die zu ermittelnden Eigenschaften der einzelnen Pulswelle.

Die Identifikation jeder einzelnen Pulswelle im pulsartigen Signalanteil erfolgt mittels einer beispielsweise durch die Steuer- und Speichereinheit mit Hilfe des hier enthaltenden Steuer- und Auswerteprogramms ausgeführten zeitlichen Ableitung des Signals. Das Ausführen der ersten zeitlichen Ableitung mit einer nachfolgenden Fußpunktbestimmung hat sich als geeignet herausgestellt, ist jedoch nicht darauf beschränkt.

Bei der hier beispielhaft beschriebenen Fußpunktmethode werden alle Wendepunkte mit einem positiven Anstieg bestimmt. Diese zeichnen sich in der ersten Ableitung gerade dadurch aus, dass an deren Stelle der Wert der ersten Ableitung extremal ist:

$$w = \arg max \frac{dP_{osc}}{dt}$$

w sind dabei die jeweiligen Positionen der "positiven" Wendepunkte. Das Ergebnis ist eine Menge aller ermittelten Ausschläge, d.h. Peaks, innerhalb des pulsartigen Signalanteils.

Anschließend werden alle Peaks der 1. Ableitung der Größe nach absteigend sortiert.

$$s = sort\left(\frac{dP_{osc}}{dt}(w)\right)$$

dabei ist s hier die sortierte Folge der ermittelten Wendepunkte.

Diese sortierte Folge s der Peaks wird schrittweise analysiert. Jeder Peak wird dann als ein Wendepunkt erfasst, wenn dessen Mindesthöhe größer als 0 ist und wenn dessen Abstand zu allen bereits erfassten Wendepunkten größer als ein festgelegtes zeitliches Intervall bezüglich einer vorgegebenen Samplerate FS ist.

Jeder Peak w(i) aus der Folge s(i) ist somit dann ein Wendepunkt idx, wenn folgende Bedingungen gleichzeitig erfüllt sind:

$$(a)\ s(i) > 0$$

und

$$(b)\; idx = \left\{ w(i) \middle| s(i) > 0 \wedge \left| \frac{w(i) - idx(j)}{FS} \right| \cdot 1000 > D, mit\; j < i \right\}$$

Die Bedingung (b) bringt zum Ausdruck, dass ein Peak $w(i)$ einen bestimmten Mindestabstand D zu einem bereits identifizierten Peak $idx(j)$ aufweisen muss. Dieser Mindestabstand ist unabhängig von der Samplerate. Hier beträgt dieser beispielsweise 350 ms.

Ein folgendes aufsteigendes Sortieren von *idx* bringt die Indizes in die richtige zeitliche Reihenfolge.

Um ein eventuelles Rauschen und geringe Störungen im Signal und erst recht bei der Differentiation zu vermeiden, kann die 1. Ableitung ggf. nach dem Verfahren von "Savitzsky-Goley" berechnet werden.

Von entscheidender Bedeutung für die Identifikation der einzelnen Pulswelle im pulsierenden Signalanteil ist die Lage ihrer jeweiligen Fußpunkte. Diese können aus den Wendepunkten heraus ermittelt werden.

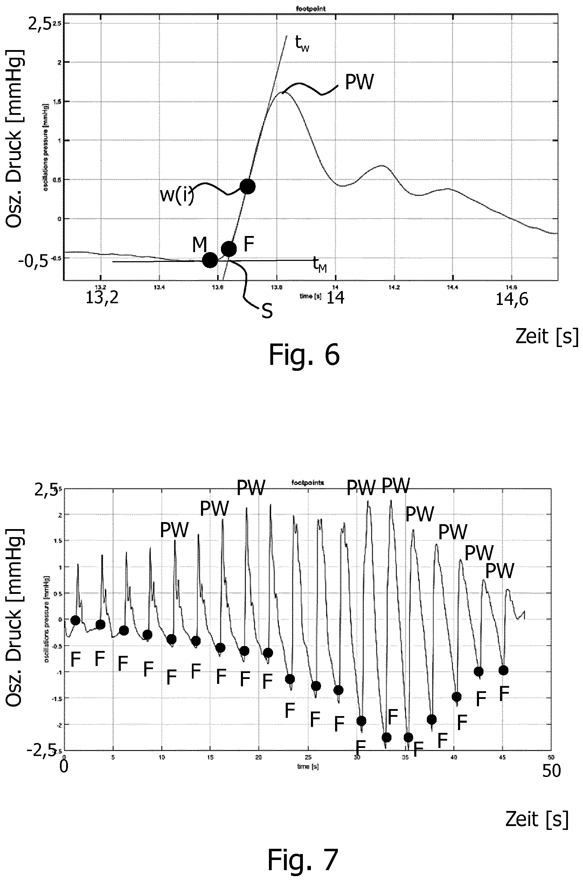

Ausgehend vom jeweils ermittelten Wendepunkt werden die Fußpunkte der einzelnen Pulswellen beispielsweise mit Hilfe sich schneidender Tangenten in der Umgebung jedes einzelnen Maximums in der 1. Ableitung des $P_{osc}$-Signals lokalisiert, d.h. also in der Umgebung des zuvor bestimmten Wendepunktes. Fig. 6 zeigt hierzu eine illustrierende Darstellung. Neben der ermittelten Tangente $t_w$ im Wendepunkt $w(i)$ wird dabei das erste lokale Minimum M im zeitlichen Bereich vor der Pulswelle gesucht. Dieses lokale Minimum M weist eine waagerechte Tangente $t_M$ auf. Anschließend wird der Schnittpunkt S zwischen der waagerechten Tangente $t_M$ und der Regressionsgeraden, d.h. der Tangente $t_w$ berechnet. Dieser Schnittpunkt S, projiziert auf das Signal, ist der Fußpunkt F der Pulswelle PW. Dieser Punkt kann zusätzlich adjustiert werden.

Für jeden Fußpunkt F mit den Koordinaten $F_j(x, y)$ gilt dabei

$$x_i = idx(j) - i \cdot gap$$

wobei unter gap die jeweilige Lücke in den Samples zu verstehen ist, und $y_i = P_{osc}(x_i)$ mit i =1,...,n die Regressionspunkte neben dem Wendepunkt $idx(j)$. Dabei ist n die Anzahl der Regressionspunkte. Basierend auf den Koordinaten $(x_i, y_i)$ der Fußpunkte $F_j$ können nun Koeffizienten *a,b* einer Geradengleichung g(x) = ax + b ermittelt werden. Fig. 7 zeigt hierzu illustrierend eine Reihe von ermittelten Fußpunkten F im Pulsanteil des Signals, die jeweils einzelne Pulswellen zwischen sich einschließen, von denen einige beispielhaft mit dem Bezugszeichen PW markiert sind.

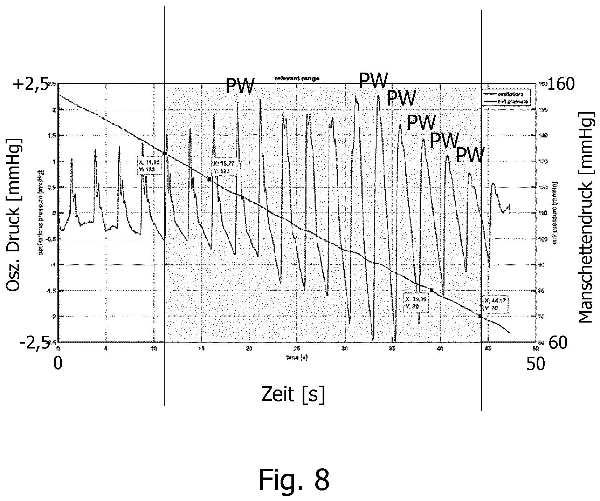

In einem nächsten Schritt wird ein relevanter Teil des oszillatorischen Signalanteils $P_{osc}$ mit den ermittelten einzelnen Pulswellen PW definiert. Fig. 8 zeigt hierzu ein illustrierendes Beispieldiagramm. Es wird hierzu von einem sinnvollen Druckwert ausgegangen, der auf den systolischen und diastolischen Blutdruckwert bezogen ist. Der Druckbereich des Pulssignals liegt insbesondere zwischen einem Druckwert von beispielsweise 10 mmHg oberhalb des systolischen Wertes und 10 mmHg unterhalb des diastolischen Druckwertes.

Eine Herzperiode wird dann als ein Abschnitt zweier aufeinanderfolgender und wie vorhergehend ermittelter Fußpunkte $F_i$ definiert. Der Pulsdruck PP einer jeden Periode wird aus der Differenz von Systole und Diastole ermittelt.

Der mittlere Druck kann über folgende Beziehung ermittelt werden:

$$y_m = \frac{1}{T} \int_0^T y\, dt \cong \frac{1}{N} \sum_{i=1}^N y(i)$$

T ist dabei die Länge des Zeitintervalls der Periode m, über das gemittelt wird, N ist entsprechend die Zahl der Samples über die gemittelt wird, wobei $y(i)$ die Amplitude jedes einzelnen registrierten Samples i ist.

Anschließend erfolgt eine Subtraktion des mittleren Drucks von den Pulsdrücken der Periode. Hierdurch lässt sich ein so genanntes zero mean-Signal erhalten.

Anschließend werden die Amplituden der einzelnen Pulswellen auf die Höhe des gemessenen Pulsdruckes PP skaliert.

Von Interesse ist jedoch nicht nur die Lage und Höhe jeder einzelnen Pulswelle, sondern auch deren Form. Diese kann wie folgt analysiert werden:

Die Pulswellen im Aufpump- und/oder im Ablassvorgang haben je nach aktuellem Manschettendruck eine unterschiedliche Form. Diese Formen können in Klassen eingeordnet werden. Jede Klasse wird dabei durch eine vorgegebene Berechnungsfunktion repräsentiert, die bestimmte Funktionsverläufe der Pulswelle wiedergibt. Es können dabei beliebig viele Berechnungsfunktionen definiert werden, deren Werte als feste Größen in der Steuer- und Speichereinheit, aber auch in den jeweils externen Auswerteeinrichtungen vorgegeben sein können. Anzahl und Ausgestaltung der jeweiligen Eigenschaften der Berechnungsfunktionen können dabei flexibel veränderbar sein.

Die Anzahl der Berechnungsfunktionen, also die Klassenanzahl, beträgt k, wobei k eine natürliche Zahl größer 0 ist.

Jede Pulswelle kann dann durch die Signalverarbeitung in der Steuer- und Speichereinheit einer derartigen Klasse zugeordnet werden. Ausschlaggebend für diese Klassen sind die unterschiedlichen Ausprägungen im Verlauf der einzelnen Pulswelle PW, beziehungsweise die Existenz von Punkten und Merkmalen einer ersten und einer zweiten Schulter des einzelnen Pulspeaks sowie eine Stärke einer Dikrotie bzw. Inzisur des jeweiligen Peaks. Dabei beschreibt die erste und zweite Schulter die Stärke der "Doppelgipfeligkeit" eines Peaks, die Dikrotie die Veränderung der Pulswelle durch das Eintreffen der reflektierten Welle, während die Inzisur die Ausprägung der Dikrotie zu einem lokalen Minimum angibt. Dikrotie und Inzesur beschreiben somit, wie "rund" die einzelne Periode, d.h. die einzelne Pulswelle, in ihrem Verlauf ist. Ausgehend davon kann jede einzelne Pulswelle in Formklassen eingeordnet werden.

Die Zuordnung zu einer Klasse kann nach dem Prinzip des "nearest neighbour" erfolgen. So kann beispielsweise die einzelne Pulswelle PW eine linksseitige Schulter aufweisen, während eine erste Berechnungsfunktion der Klasse k = 1 keine linksseitige Schulter vorgesehen hat und eine zweite Berechnungsfunktion der Klasse k = 2 eine linksseitige Schulter aufweist. Die einzelne Pulswelle weicht somit von der schulterlosen Berechnungsfunktion in ihrer Form stärker ab als von der Berechnungsfunktion mit der linksseitigen Schulter. Somit gehört die Pulswelle PW zur Klasse k =2 und wird somit durch die Berechnungsfunktion der Klasse k = 2 repräsentiert.

Eine weitere Möglichkeit die Zuordnung zu einer Klasse vorzunehmen ist, die Ähnlichkeitsanalyse mit Hilfe einer dynamische Zeitentzerrung durchzuführen, um die Periodenlängen und Zeiten invariant zu gestalten und einen Vergleich zu ermöglichen. Durch diesen Vergleich werden die Formen der Pulswellen miteinander und gegenüber den Berechnungsfunktionen vergleichbar und den Klassen zuordenbar.

Für jede dieser Klassen kann ein klassenspezifischer Algorithmus angegeben werden, welcher die hämodynamischen Parameter für die Klasse bestimmt. Eine Transferfunktion, bestehend aus einer Menge von jeweils zwei Teilfunktionen hat sich als geeignet herausgestellt, ist jedoch nicht darauf beschränkt. Diese Teilfunktionen beschreiben die Verstärkung des Amplitudenspektrums und die Verschiebung des Phasenspektrums. Zur Ermittlung des transformierten Signals wird von dem Signal das Amplituden- und Phasenspektrum ermittelt. Hierzu wird beispielweise eine Fast Fourier Transformation (FFT) angewendet.

Das hieraus gewonnene Amplitudenspektrum wird mit der Verstärkung der Transferfunktion multipliziert. Das Phasenspektrum wird mit der Phasenverschiebung addiert. Durch die Rücktransformation erhält man die aortale Pulskurve dieser spezifischen Klasse. Abschließend erfolgt ein Auswerten der transformierten Daten zum Ermitteln hämodynamischer Parameter.

Um Unstetigkeiten bei der Transformation zu vermeiden, werden die Zwischenbereiche zwischen den Klassen glatt gehalten. Das bedeutet, dass dann, wenn eine Pulswelle beispielsweise zwei Klassen zugeordnet werden kann, jede dieser beiden Klassen als gleichwahrscheinlich behandelt wird.

Liegt nun eine weitere Pulswelle PW ein wenig näher an einer dieser Klassen, so ist die Zuordnung eindeutig. Für einen Koeffizient $\tilde{c}$ gilt dann:

$$\tilde{c} = \alpha c_i + (1 - \alpha)c_{i+1} \ , \alpha = [0,1]$$

Dabei sind $c_i$ und $c_{i+1}$ die Koeffizienten beiden nächsten Nachbarn.

Die Glattheit gewährleistet, dass die beiden Perioden nach der Transformation nicht gänzlich verschieden sind, egal zu welcher Klasse die erste Periode zugeordnet wurde.

Nach der Transformation liegt für jede skalierte, am Arm oder einer anderen Extremität bestimmten Pulswelle eine aortale Pulswelle vor mit ihren jeweils spezifischen hämodynamischen Parametern. Diese Informationen sind entsprechend gewichtet zu mitteln. Es hat sich als geeignet herausgestellt, an den einzelnen aortalen Pulswellen bestimmte Parameter $\bar{x}$ zusammen mit einem zugehörigen Konfidenzintervall $\tilde{x}$ zu ermitteln, es ist jedoch nicht darauf beschränkt. Das Konfidenzintervall $\tilde{x}$ zu $x$ ergibt sich wie folgt:

$$\tilde{x} = \bar{x} \pm 1{,}96 \frac{\sigma}{\sqrt{N}}$$

Der Parameter $\sigma$ ist hier die Standardabweichung und N ist die Anzahl der Perioden. Mit steigenden N verkleinert sich das Konfidenzintervall, eine langsame Aufpump- als auch Ablassgeschwindigkeit, sowie eine im Vergleich dazu schnelle Herzrate erhöhen die Genauigkeit. Bei niedrigen Herzraten sollten somit die Geschwindigkeiten der Aufpumpphase sowie der Ablassphase entsprechend angepasst werden.

Die Breite des 95% Konfidenzintervalls gibt Auskunft über die Zuverlässigkeit und Güte des Parameters. Anhand der extrahierten am Arm oder einer anderen Extremität bestimmten Pulswellen und der errechneten aortalen Pulswellen ist es möglich, weitere hämodynamische Parameter, wie z.B. aortaler (zentraler) Blutdruck, für eine umfangreiche Pulswellenanalyse zu ermitteln.

Das erfindungsgemäße Verfahren und die zur Durchführung dieses Verfahrens dienende Anordnung wurden anhand von Ausführungsbeispielen näher erläutert. Weitere Ausführungsformen und Ausgestaltungen ergeben sich aus den Unteransprüchen und im Rahmen fachmännischen Handelns.

Bezugszeichenliste

| | |
|---|---|
| 1 | Manschette |
| 2 | Pumpe |
| 2a | Schlauch |
| 3 | Drucksensor |
| 4 | Steuer- und Speichereinheit |
| 5 | Steuer- und Auswerteprogramm |
| 6 | externes Speichermittel |
| 7 | Schnittstelle |
| 8 | weitere Schnittstelle |
| 9 | externe Auswerteeinheit |
| 10 | externes Auswerteprogramm |
| 11 | Display |
| Inf | Aufpumpphase |
| Def | Ablassphase |
| $P_{meas}$ | gemessener zeitlicher Druckverlauf |
| $P_{cuff}$ | Manschettendruck |
| $P_{osc}$ | oszillatorischer Anteil des Druckes |
| F | Fußpunkt |
| $t_w$ | Wendepunkttangente |
| $t_M$ | Tangente in Minimum |

**Patentansprüche**

**1.**  Verfahren zum Betreiben einer Blutdruckmessvorrichtung mit folgenden Verfahrensschritten:

- Ausführen einer Blutdruckmessung mit der Blutdruckmessvorrichtung, bestehend aus einer Manschette (1) zum Umlegen um eine Extremität, einer Pumpe (2) zum Aufpumpen der Manschette und einem Drucksensor (3) zum Registrieren des Drucks innerhalb der Manschette, wobei ein Aufpumpen der Manschette in einer Aufpumpphase (Inf) und ein Ablassen des Drucks in der Manschette in einer Ablassphase (Def) erfolgt,
- Registrieren und Speichern des zeitlichen Druckverlaufs ($P_{meas}$) in der Manschette (1) während der Aufpumpphase und/oder während der Ablassphase in einer Steuer- und Speichereinheit (4),
- Extrahieren eines pulsartigen Signalanteils ($P_{osc}$) aus dem zeitlichen Druckverlauf des gemessenen Manschettendrucks während der Aufpumpphase und/oder der Ablassphase durch eine Signalanalyse innerhalb der Steuer- und Speichereinheit (4),
- Signalanalyse des extrahierten pulsartigen Signalanteils ($P_{osc}$) innerhalb der Steuer- und Speichereinheit mit den Schritten:
- Zerlegen des pulsartigen Signalanteils in Daten über Einzelperioden zum Identifizieren von einzelnen Pulswellen (PW),
- Kombinieren der Daten der jeweiligen einzelnen Pulswellen (PW) aus Werten mindestens einer Berechnungsfunktion aus einer vorgegebenen Berechnungsfunktionsmenge zum Erzeugen einer jeweiligen klassifizierten einzelnen Pulswelle (PW),
- Transformieren der jeweiligen klassifizierten einzelnen Pulswelle,
- Auswerten der transformierten Daten zum Ermitteln hämodynamischer Parameter.

**2.**  Verfahren nach Anspruch 1,
**dadurch gekennzeichnet, dass**
das Zerlegen des pulsartigen Signalanteils in Daten über die einzelnen Pulswellen (PW) im zeitlichen Verlauf des pulsartigen Signalanteils (P_osc) erfolgt.

3. Verfahren nach Anspruch 1,
   **dadurch gekennzeichnet, dass**
   bei dem Kombinieren der Daten der jeweiligen einzelnen Pulswellen (PW) zum Erzeugen der jeweiligen klassifizierten einzelnen Pulswelle mindestens zwei Berechnungsfunktionen gewichtet miteinander kombiniert werden.

4. Verfahren nach Anspruch 1 oder 3,
   **dadurch gekennzeichnet, dass**
   das Transformieren der jeweiligen klassifizierten einzelnen Pulswelle mittels einer Fourier-Analyseeinheit in das periodenspezifische Amplituden- und Phasenspektrum erfolgt.

5. Verfahren nach einem der vorhergehenden Ansprüche,
   **dadurch gekennzeichnet, dass**
   eine Rücktransformation des periodenspezifischen Amplituden- und Phasenspektrums der klassifizierten einzelnen Pulswelle zum Bestimmen des Zeitverlaufs der aortalen Pulswelle erfolgt.

6. Verfahren nach Anspruch 1,
   **dadurch gekennzeichnet, dass**
   das Registrieren des zeitlichen Druckverlaufs in der Aufpumpphase und/oder in der Ablassphase digital mit einer festen gerätespezifischen Abtastrate und einer festen gerätespezifischen Auflösung erfolgt, wobei ein anschließendes Sampeln auf eine plattformunabhängige Abtastrate in Verbindung mit einer Approximation des gemessenen zeitlichen Druckverlaufs (P_meas) in der Manschette erfolgt.

7. Verfahren nach einem der Ansprüche 1 oder 6,
   **dadurch gekennzeichnet, dass**
   bei dem Extrahieren des pulsförmigen Signalanteils (P_osc) über einen Bandpass-Filter ein Abseparieren eines nichtoszillierenden Manschettendrucks (P_cuff) erfolgt.

8. Verfahren nach einem der vorhergehenden Ansprüche,
   **dadurch gekennzeichnet, dass**
   der abseparierte nichtoszillierende Manschettendruck (P_cuff) auf Übereinstimmung mit einem Normverlauf geprüft wird, wobei aus dem Grad der Abweichung des nichtoszillierenden Pumptrends von dem Normverlauf ein Bestimmen eines Artefaktparameters zum Anzeigen einer messwertverfälschenden Beeinflussung erfolgt.

9. Verwenden eines Verfahrens nach einem der Ansprüche 1 bis 8 zum Ermitteln und Analysieren einer aortalen Pulswelle und zur Bestimmung hämodynamischer Parameter der Pulswellenanalyse, insbesondere einem aortalen Blutdruck.

**Claims**

1. A method for operating a blood pressure measuring device by means of the following method steps:

   - executing a blood pressure measurement by means of the blood pressure measuring device composed of a cuff (1) for being placed around an extremity, a pump (2) for inflating the cuff, and a pressure sensor (3) for registering the pressure within the cuff, wherein inflating of the cuff is performed in an inflating phase (Inf), and deflating of the pressure within the cuff is performed in a deflating phase (Def),
   - registering and storing the temporal pressure course (P_meas) within the cuff (1) during the inflating phase and/or during the deflating phase in a control and storage unit (4),
   - extracting a pulse-like signal component (P_osc) from the temporal pressure course of the measured cuff pressure during the inflating phase and/or the deflating phase by a signal analysis within the control and storage unit (4),
   - signal analysis of the extracted pulse-like signal component (P_osc) within the control and storage unit by means of the following steps:
     - decomposing of the pulse-like signal component into data about individual periods for identifying individual pulse waves (PW),
     - combining the data of the respective individual pulse waves (PW) from values of at least one calculation function of a predefined calculation function amount for generating a respective classified individual pulse wave (PW),
- transforming the respective classified individual pulse wave,
- evaluating the transformed data for ascertaining hemodynamic parameters.

2. The method according to claim 1,
**characterized in that**
the decomposing of the pulse-like signal component into data about the individual pulse waves (PW) is performed in the temporal course of the pulse-like signal component ($P_{osc}$).

3. The method according to claim 1,
**characterized in that**
in combining the data of the respective individual pule waves (PW) for generating the respective classified individual pulse wave, at least two calculation functions are combined with one another in a weighted way.

4. The method according to claim 1 or 3,
**characterized in that**
the transforming of the respective classified individual pulse wave into the period-specific amplitude and phase spectrum is performed by means of a Fourier analysis unit.

5. The method according to any one of the preceding claims, **characterized in that**
a retransformation of the period-specific amplitude and phase spectrum of the classified individual pules wave is performed for determining the temporal course of the aortal pulse wave.

6. The method according to claim 1,
**characterized in that**
the registering of the temporal pressure course is performed in the inflating phase and/or the deflating phase in a digital way with a fixed device-specific sampling rate and a fixed device-specific resolution, wherein a subsequent sampling to a platform-independent sampling rate is performed in conjunction with an approximation of the measured temporal pressure course ($P_{meas}$) in the cuff.

7. The method according to claim 1 or 6,
**characterized in that**
in extracting the pulse-like signal component ($P_{osc}$) over a bandpass filter, separating of a non-oscillating cuff pressure ($P_{cuff}$) is performed.

8. The method according to any one of the preceding claims, **characterized in that**
the separated non-oscillating cuff pressure ($P_{cuff}$) is checked for compliance with a standard course, wherein from the degree of deviation of the non-oscillating pumping trend from the standard course, determining an artefact parameter is performed for indicating an influence falsifying the measured value.

9. Use of a method according to any one of claims 1 to 8 for ascertaining and analyzing an aortal pulse wave and for determining hemodynamic parameters of the pulse wave analysis, in particular an aortal blood pressure.


**Revendications**

1. Procédé pour faire fonctionner un dispositif de mesure de la pression artérielle, comprenant les étapes de procédé suivantes consistant à :

   - effectuer une mesure de la pression artérielle avec le dispositif de mesure de la pression artérielle, constitué d'un brassard (1) destiné à être placé autour d'un membre, d'une pompe (2) destinée à gonfler le brassard, et d'un capteur de pression (3) destiné à enregistrer la pression à l'intérieur du brassard, un gonflage du brassard étant effectué dans une phase de gonflage (Inf) et un dégonflage de la pression dans le brassard étant effectué dans une phase de dégonflage (Def),
   - enregistrer et mémoriser dans une unité de commande et de mémorisation (4) l'évolution temporelle de la pression ($P_{meas}$) dans le brassard (1) pendant la phase de gonflage et/ou pendant la phase de dégonflage,
   - extraire une composante de signal ($P_{osc}$) de type impulsionnel de l'évolution temporelle de la pression mesurée dans le brassard pendant la phase de gonflage et/ou la phase de dégonflage par une analyse de signal à l'intérieur de l'unité de commande et de mémorisation (4),
- analyser la composante de signal extraite ($P_{osc}$) de type impulsionnel à l'intérieur de l'unité de commande et de mémorisation avec les étapes consistant à :
- décomposer la composante de signal de type impulsionnel en données sur des périodes individuelles pour identifier des ondes de pouls individuelles (PW),
- combiner les données des ondes de pouls individuelles respectives (PW) à partir de valeurs d'au moins une fonction de calcul d'un ensemble de fonctions de calcul prédéterminé pour générer une onde de pouls individuelle classifiée respective (PW),
- transformer l'onde de pouls individuelle classifiée respective,
- évaluer les données transformées pour déterminer des paramètres hémodynamiques.

2. Procédé selon la revendication 1,
**caractérisé en ce que**
la décomposition de la composante de signal de type impulsionnel en données sur les ondes de pouls individuelles (PW) s'effectue pendant l'évolution temporelle de la composante de signal ($P_{osc}$) de type impulsionnel.

3. Procédé selon la revendication 1,
**caractérisé en ce que**
lors de la combinaison des données des ondes de pouls individuelles respectives (PW) pour générer l'onde de pouls individuelle classifiée respective, au moins deux fonctions de calcul sont combinées l'une avec l'autre de manière pondérée.

4. Procédé selon la revendication 1 ou 3,
**caractérisé en ce que**
la transformation de l'onde de pouls individuelle classifiée respective est effectuée au moyen d'une unité d'analyse de Fourier dans le spectre d'amplitude et de phase spécifique à la période.

5. Procédé selon l'une des revendications précédentes,
**caractérisé en ce que**
une transformation inverse du spectre d'amplitude et de fréquence spécifique à la période de l'onde de pouls individuelle classifiée est effectuée pour déterminer l'évolution temporelle de l'onde de pouls aortique.

6. Procédé selon la revendication 1,
**caractérisé en ce que**
l'enregistrement de l'évolution temporelle de la pression dans la phase de gonflage et/ou dans la phase de dégonflage est effectué de manière numérique avec un taux d'échantillonnage fixe spécifique à l'appareil et avec une résolution fixe spécifique à l'appareil, puis un échantillonnage à un taux d'échantillonnage indépendant de la plate-forme est effectué en liaison avec une approximation de l'évolution temporelle de la pression mesurée ($P_{meas}$) dans le brassard.

7. Procédé selon l'une des revendications 1 ou 6,
**caractérisé en ce que**
une séparation d'une pression de brassard ($P_{cuff}$) non oscillante est effectuée lors de l'extraction de la composante de signal ($P_{osc}$) de type impulsionnel par l'intermédiaire d'un filtre passe-bande.

8. Procédé selon l'une des revendications précédentes,
**caractérisé en ce que**
la pression de brassard ($P_{cuff}$) non oscillante séparée est contrôlée quant à une concordance avec une évolution normalisée, et le degré de l'écart de la tendance de pompage non oscillante par rapport à l'évolution normalisée est utilisé pour déterminer un paramètre d'artefact afin d'indiquer une influence faussant la valeur de mesure.

9. Utilisation d'un procédé selon l'une des revendications 1 à 8 pour déterminer et analyser une onde de pouls aortique et pour déterminer des paramètres hémodynamiques de l'analyse de l'onde de pouls, en particulier une pression artérielle aortique.

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20170181648 A1
- DE 102004011779 B4
- US 2013289421 A1
- US 5533511 A
- WO 9203966 A1